(19) Europäisches Patentamt

European Patent Office                    (11) Publication number:          **0 210 152**

Office européen des brevets                                              **A2**

(12)                    **EUROPEAN PATENT APPLICATION**

(21) Application number: 86850251.9          (51) Int. Cl.⁴: **A61C 8/00**

(22) Date of filing: 11.07.86

(30) Priority: 24.07.85 SE 8503579          (71) Applicant: **The Institute for Applied**
24.07.85 SE 8503581                      **Biotechnology**
**Box 33053**
(43) Date of publication of application:     **S-400 33 Göteborg(SE)**
**28.01.87 Bulletin 87/05**
(72) Inventor: **Branemark, Per-Ingvar**
(84) Designated Contracting States:          **Ändergatan 3**
**AT BE CH DE FR GB IT LI LU NL**         **S-431 39 Mölndal(SE)**

(74) Representative: **Barnieske, Hans Wolfgang**
**c/o H.W. Barnieske Patentbyrå AB P.O. Box**
**25 Turingegatan 26**
**S-151 21 Södertälje 1(SE)**

(54) A device for securing a prosthesis.

㊿ The present invention relates to a device for securing a prosthesis, comprising a first attachment means implanted in the body and a second attachment means secured to the prosthesis, wherein the first attachment means comprises at least two attachment elements (3, 13) implanted in the body, the upper parts thereof being provided with first positioning means and a splint (4, 14) anchored thereto by means of second positioning means (6, 16), the second attachment means comprising two or more attachment elements (7, 17) intended to cooperate with said splint (4, 14).

FIG.2

The present invention relates to a device for securing a prosthesis, comprising a first attachment means implanted in the body and a second attachment means secured to the prosthesis.

Devices for securing prostheses, such as an ear prosthesis, protuding externally from the body, or a dental prosthesis, have not been successful so far.

Within the field of odontologicial prosthetics a method has long seen sought to enable permanent anchoring of prostheses. This is primarily because in many cases of partial or total loss of teeth, these is some difficulty in achieving satisfactory prosthesis retention using conventional methods. This is particularly so if psychological or professional factors complicate or prevent the use of removable prostheses.

Fixed prostheses are therefore required as well as conventional removable prostheses. This is also evident from the numerous methods which have been tried all over the world for permanently securing dental prostheses. Special types of implants have been used and these can be divided into two main groups. The first group constitutes implants in the form of more or less finely meshed net or a metal skeleton which is applied on the alveolar bone, either in one piece or in separate sections. In the second group the implants comprise various types of screws or pins which are anchored in the alveolar bone. Combinations of these two main groups also exist and the material used for the implants is normally stainless steel, chromium, cobalt, molybdenum alloys, etc.

However, the known implants do not function satisfactorily, many of them working loose after a short time and also causing inflammation.

A method has, however, been developed for permanently anchoring individual teeth in the alveolar bone. Briefly, this method entails implanting a screw, made of titanium or titanium alloy, in a hole drilled in the bone so that the head of the screw is level with or just below the surface of the alveolar bone. The screw is then covered with a piece of mucous membrane and then left to rest for a certain period (3 -6 months) to allow the bone to grow around and form a firmly bonded unit with the implanted screw. After the rest period the screw is exposed and a spacer arranged thereon, after which a single tooth prosthesis is anchored to the spacer.

A plurality of teeth can also be attached if at least two of the original teeth remain. These teeth are then cut and a hole drilled on the root side to enable a root pillar of gold to be cemented in. A bridge or "skeleton" of gold is then welded together between the teeth.

This conventional method has also been used for implants, a wax model being prepared first and then embedded in a mould, after which the wax is melted and replaced by a gold alloy. However, this method is time-consuming and expensive, as well as it being difficult to achieve the necessary precision with respect to the anchoring means. The anchoring means have therefore been unevenly loaded causing them to work loose after some time in use.

A known method of securing an ear prosthesis to the body comprises a press-stud connection in which one connection element is arranged at the end of a stud and the other connection element is permanently secured to the ear. The first stud is implanted in the cranium and is allowed to become embedded there over a certain healing period, whereupon the ear is fitted to the stud with the aid of the second connection element. There are a number of draw backs to the device. The protruding stud is easily knocked, for instance, causing it to become dislodged from the skull during the healing process or causing the stud to become embedded incorrectly in the cranium. Even if the stud becomes satisfactorily embedded in the cranium, there remain drawbacks with this type of connection. If, for some reason, the connection elements are unintentionally disengaged, the wearer will lose the ear prosthesis. Furthermore, the press-stud connection does not prevent the ear prosthesis from turning on the skull. It is thus difficult to apply the prosthesis in the correct position on the cranium and the prosthesis is easily turned from its correct position by an external force.

The object of the present invention is to eliminate the above-mentioned drawbacks of conventional methods of securing a prosthesis and to achieve a device of the type described in the introduction which will detachably position a prosthesis in the correct position, without risk of the prosthesis being lost or twisted during normal use.

This is achieved by the device according to the present invention substantially in that the first attachment means comprises at least two attachment elements implanted in the body, the upper parts thereof being provided with first positioning means and a splint anchored thereto by means of second positioning means, the second attachment means comprising two or more attachement elements intended to cooperate with said splint.

Thus by implanting two or more attachment elements, provided with an upper part containing first positioning means, in the body, in such a way that the upper parts of the attachment elements are lying substantially level with or protruding negligibly above the surface of the body, leaving the attachment elements to become firmly embedded in the body. A splint is anchored to the implanted attachment elements by means of second positioning means engaging in the first positioning means, the prosthesis being provided with attachment elements to fit the splint and finally securing the attachment elements to the splint.

Since the upper surfaces of the attachment elements are substantially countersunk in the body, they are not so susceptible to external forces as the known attachment elements. Any knocks to which they are subjected will be considerably less and the attachments will not become dislodged as easily or be shifted from the intended position. The prosthesis is correctly positioned on the splint at several attachment points and cannot therefore be twisted. Furthermore, the prosthesis will remain should one of the attachment elements become dislodged from the splint.

The attachment means suitably comprise U-shaped clips or the like which are attached by snapping in over said splint.

The splint is preferably curved and is removably screwed to the attachment elements by means of screws.

The invention will be described in more detail in the following, with reference to the accompanying drawings, in which

Figure 1 shows an exploded view of a first embodiment of a device according to the invention, and

Figure 2 shows an exploded perspective view of a second embodiment of a device according to the invention.

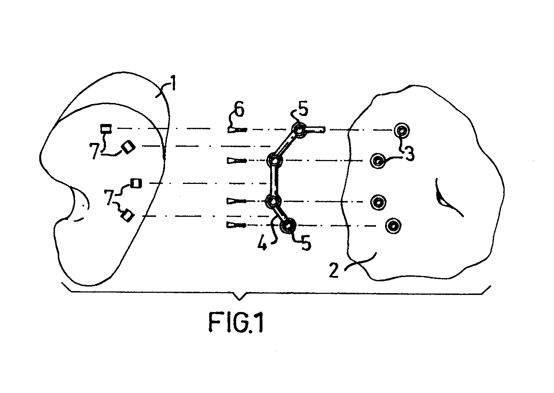

Figure 1 shows an ear prosthesis 1 provided with a second attachment means in the form of attachment elements 7 for securing the ear prosthesis 1 to a first attachment means consisting of attachment elements 3 implanted in a cranium 2. A splint 4 between two or more attachment elements 3 provides an attachment surface for the elements 7. The splint 4 is secured to the attachment elements 3 by means of positioning means 6, preferably screwed into the attachment elements 3 through holes 5.

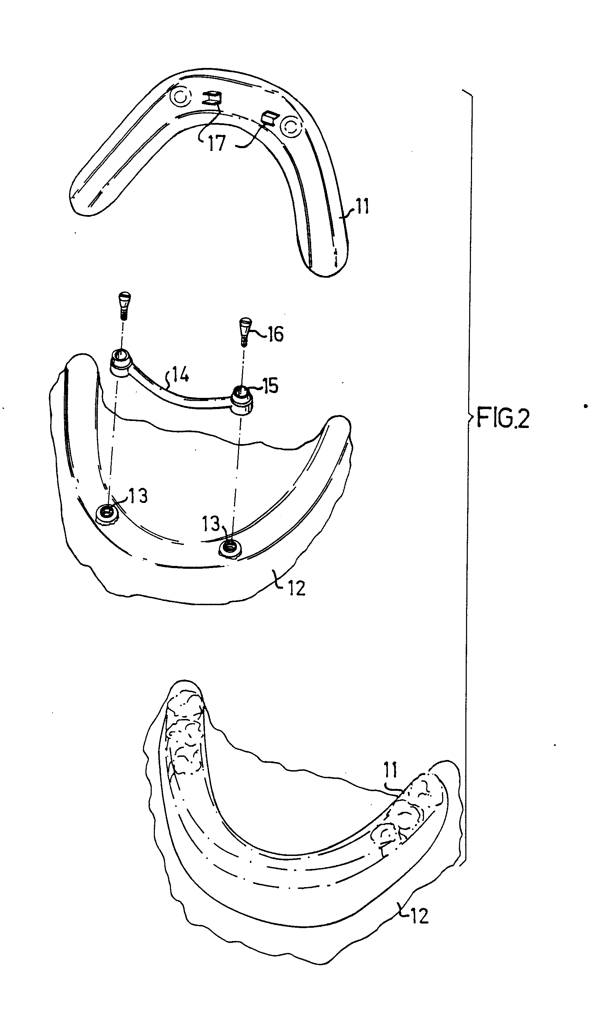

Figure 2 shows a device for securing a plurality of teeth to at least two attachment elements 13 implanted in the alveolar bone 12. The means also includes two or more attachment means 17 arranged on a prosthesis 11, on the surface facing the jaw, and a splint 14 which is removably screwed to the attachment elements by means of screws 16 passed through holes 15 in the splint 14 and screwed into the threaded holes in the attachment elements 13, or is fixed thereto by glueing, welding or the like.

The artificial ear 1, according to figure 1, consisting of a suitable plastic material for instance, which is to be secured to the cranium 2, is provided with attachment elements 7. These may consist of U-shaped clips, preferably of metal, magnetic plates or the like. The attachment elements 3, having external screw-threading, are screwed into holes in the cranium 2 so as to lie substantially level with or protruding negligibly above the surface of the skull. The attachment elements 3 are then allowed to become embedded into the skull over a healing period. Thereafter the splint 4 is secured by screws 6 to the attachment elements 3 which are provided for the purposes with internally threaded holes. The splint 4 is provided with through-holes 5 for the screws 6, corresponding to the number of attachment elements 3. When screwed in the heads of the screws 6 are countersunk in the splint 4. The splint 4 consists of a metal strip suitable for snap-in function, a magnetic strip or the like. Finally, the ear prosthesis 1 with its second attachment means is pressed against the first attachment means on the cranium 2 so that the clip grips the splint 4 or so that the respective magnets are attracted.

The device, according to figure 2, is preferably made of titanium or titanium alloy, as are also the attachment elements 13. The attachment means 17 consist of U-shaped clips or the like, which are snapped in over the splint 14. The splint 14 preferably has round cross section or some other cross section suitable for snap-on attachment. To achieve favourable loading of the attachment elements 13, the splint 14 is preferably curved to fit the shape of the jaw. Furthermore, a resilient member may be arranged in the force-transmitting path between prosthesis 11 and attachment elements 13. The resilient member may consist of a ring of silicon, rubber or the like, and be arranged between each attachment element 13 and the splint 14. There is thus limited movement between splint 14 and attachment elements 13, corresponding spaces for the screws 16 being provided in the prostheses 11. A certain flexibility can also be achieved, of course, through the shape of attachment means 17 or at the point where they are secured to the prosthesis 11.

The present invention thus enables essentially permanent anchoring of an ear prosthesis and a dental prosthesis respectively which, however, can easily be removed for cleaning or the like. The attachment elements are of course made of a material compatible with body tissue, such as titanium. Furthermore, the device can also be used for securing other prostheses externally to the body, such as eyebrows or noses. Similarly, the design of clips or splint may be varied to give more or less permanent attachment. A combination of snap-in and magnetic attachment is also possible.

The invention is not limited to the embodiment shown in the drawings. It can be modified in many ways within the scope of the following claims.

## Claims

1. A device for securing a prosthesis, comprising a first attachment means implanted in the body and a second attachment means secured to the prosthesis, wherein the first attachment means comprises at least two attachment elements (3, 13) implanted in the body, the upper parts thereof being provided with first positioning means and a splint (4, 14) anchored thereto by means of second positioning means (6, 16), the second attachment means comprising two or more attachment elements (7, 17) intended to cooperate with said splint (4, 14).

2. A device according to claim 1, wherein the attachment means (7, 17) comprise U-shaped clips or the like which are attached by snapping in over said splint (4, 14).

3. A device according to claim 2, wherein the splint (4, 14) is curved.

4. A device according to claim 3, wherein the splint (4, 14) is removably screwed to the attachment elements (3, 13) by means of screws (6, 16).

5. A device according to claim 4, wherein the splint (4, 14) for each attachment element (3, 13) is provided with a hole (5, 15), the first attachment means comprises screw-threaded holes in the attachment elements (3, 13), and the second positioning means comprises screws (6, 16) provided with heads, the screws being passed through the holes (5, 15) in the splint (4, 14) and screwed into the threaded holes in the attachment elements (3, 13).

6. A device according to claim 3, wherein the splint (4, 14) is secured to the attachment elements (3, 13) by means of glueing, welding or the like.

7. A device according to any of the preceding claims, wherein a resilient member is arranged in the force-transmitting path between the prosthesis - (1, 11) and the attachment elements (3, 13).

8. A device according to claim 2, wherein the attachment elements (7, 17) and/or splint (4, 14) consist of magnets, effecting magnetic attraction therebetween.

FIG.1

FIG.2